Europäisches Patentamt

European Patent Office

Office européen des brevets

(19)

(11) Publication number: **0 288 737**

**A1**

(12) **EUROPEAN PATENT APPLICATION**

(21) Application number: 88104630.4

(51) Int. Cl.4: **C12Q 1/68** , **G01N 33/546**

(22) Date of filing: 23.03.88

(30) Priority: 01.04.87 US 33399

(43) Date of publication of application:
02.11.88 Bulletin 88/44

(84) Designated Contracting States:
AT BE CH DE ES FR GB IT LI LU SE

(71) Applicant: **MILES INC.**
**1127 Myrtle Street**
**Elkhart Indiana 46514(US)**

(72) Inventor: **Carrico, Robert J.**
**54256 Silver Street**
**Elkhart, IN 46514(US)**
Inventor: **Patterson, William L.**
**208 W. Beardsley**
**Elkhart, IN 46514(US)**

(74) Representative: **Adrian, Albert, Dr. et al**
**c/o BAYER AG Konzernverwaltung RP**
**Patentabteilung**
**D-5090 Leverkusen 1, Bayerwerk(DE)**

(54) **Rapid hybridization assay using latex-immobilized probe.**

(57) Nucleic acid hybridization assays are performed by using probes that are immobilized on discrete, water suspensible microparticles (latex). Assay times under 30 minutes from the initiation of hybridization through measurement of the generated signal are obtained. The methods are based on detection of hybrids through the use of labeled anti-hybrid antibody reagents or by labeling the sample nucleic acids. The assay formats are convenient as well as rapid. The use of latex supports for the probe eliminates the problems of immobilizing sample nucleic acids, allows the use of excess probe, and enables rapid and simple separation of hybrids for detection purposes.

EP 0 288 737 A1

## RAPID HYBRIDIZATION ASSAY USING LATEX-IMMOBILIZED PROBE

### BACKGROUND OF THE INVENTION

This invention relates to nucleic acid hybridization assay methods and reagent systems for detecting specific polynucleotide sequences. In particular, the invention concerns hybridization assays which can be performed in relatively short times, e.g., under 30 minutes from initiation of hybridization through measurement of the signal generated, and following convenient protocols.

Nucleic acid hybridization assays are based on the very specific base pairing between the polynucleotide sequence of interest and a complementary probe polynucleotide reagent. Such assays are useful as an analytical method in the fields of human and veterinary medicine, agriculture, and food science, among others. In particular, the method can be used to detect and identify etiological agents such as bacteria and viruses, to screen microbes for antibiotic resistance, and to detect malignant cells.

A number of years have now passed since the realization that nucleic acid hybridization offers a potentially very useful basis for assays of commercial interest. In that time, a large number of advancements and improvements have been made towards lessening the complexities and time consuming nature of the conventional procedures that had been used in the field, principally in basic research applications. Despite these very dedicated efforts, the currently best known methods remain undesirably cumbersome and still require a number of hours for completion. To have a major commercial impact, it is generally felt that the overall time of the assay must be decreased to no longer than about 30 minutes.

The principal hybridization methods known today are based either upon immobilization of the sample or probe polynucleotide or upon hybridization of both strands while in solution followed by some selective method of separating hybridized and unhybridized probe. Immobilization of the sample nucleic acids is plagued with a number of shortcomings, particularly the significant problems of the cumbersome and time consuming procedures involved and the difficulty of controlling nonspecific adsorption of interfering substances from the normally quite heterogeneous samples. The introduction of assay formats that permit the use of immobilized probes has in principle solved these particular problems but prior to the present invention have not allowed the performance of assays in less than a few hours.

Solid supports most commonly used for immobilization of nucleic acids for hybridization are microporous membranes composed of nitrocellulose or nylon. The hybridization is typically conducted with the membrane immersed in a hybridization liquid containing the complementary nucleic acid sequence. Although the microporous membranes can have high surface area, most of the surface is internal pores and is not in close contact with the bulk of the hybridization liquid; therefore, diffusion paths for the nucleic acid are long and hybridization rates are slow. Following hybridization, excess probe nucleic acid is removed from the membrane by immersion in wash solution. A significant amount of time is required to allow diffusion of the nucleic acid from inside the pores. If additional reagents such as enzyme labels are used to detect hybrids, these must be given time to diffuse into the pores and then further time is required to remove excess label in wash steps.

In-solution nucleic acid hybridizations have been conceived because it was recognized that such hybridizations are faster than in two phase systems since the soluble nucleic acid does not have to diffuse to a solid surface for hybridization to occur. Following hybridization, the hybridized and unhybridized labeled probe can be separated with hydroxylapatite which selectively binds the hybrids. However, the hydroxylapatite method has been applied only to radiolabeled probes because hydroxylapatite also binds many nonradioactive labels. In addition, the selectivity for single stranded versus hybridized probe is not absolute; therefore, increasing the level of probe gives increasing background signal in the absence of hybrid formation. As a result, the labeled probe cannot be used in large excess to accelerate the hybridization rate. Thus, hours are still required for the assay to be completed.

Sandwich hybridization has also been studied extensively because it does not require immobilization of sample nucleic acids. It employs an immobilized probe and a soluble labeled probe which hybridize to different segments of the sample strand. However, this method requires longer hybridization because three nucleic acid strands must come together to form detectable hybrids.

The use of immobilized or immobilizable probes as a way of simplifying hybridization assays has been proposed. In one approach, the resulting immobilized hybrids are detected by binding of labeled anti-hybrid antibody reagent (see European Patent Publication 163,220). Suggested solid supports for the probe include microparticles, beads, membranes, and the like, with the specifically exemplified methods using large, water insuspensible particles that yielded assay time of a few hours. In another approach, the sample nucleic acids themselves are first labeled, e.g., by contact with a labeled photochemically reactive DNA intercalating agent, and after separation, the resulting labeled hybrids are detected (see European Patent Application No. 87 102 577.1, filed February 24, 1987 and assigned to Molecular Diagnostics, Inc., West Haven, CT, USA). Again, the particular selection of supports has kept assay times above a few hours.

There is thus a well-focused and unsatisfied need for a nucleic acid hybridization format that will enable the performance of assays in under an hour, particularly under the commercial goal of 30 minutes, without sacrificing convenience or assay performance. A further advantage would be the ability to use a nonradioisotopic detection method, particularly a visual or spectrophotometric method.

The use of microparticles, including latex, has been proposed in the literature as a solid phase for separating and detecting hybrids. Noyes and Stark (1975) Cell 5:301 covalently coupled SV40 DNA to diazotized $m$-aminobenzyloxymethylcellulose particles 0.5 to 1.0 $\mu$m in diameter. Hybridization with $^3$H-labeled probe required about 24 hours for completion. European Patent Publication 154,505 suggests that solid phases including latex particles can be used for immobilization of DNA probes for use in sandwich hybridization. Means for covalently immobilizing the DNA to such particles and the performance of the product in actual assays are not discussed. The use of latex particles, principally as labels, in sandwich hybridization is also alluded to in European Patent Publication 159,719. PCT Publication WO 86-03782 describes the immobilization of DNA on crosslinked macroporous cellulosic resin particles of 40 to 60 $\mu$m diameters. Incubation periods of 2 to 4 hours were required for completion of a sandwich hybridization.

SUMMARY OF THE INVENTION

The present invention now provides nucleic acid hybridization assays which can be completed in 30 minutes or less from initiation of hybridization to measurement of generated signal. Hybridization assays based on the use of immobilized probe are significantly improved by the use of discrete, water suspensible microparticles, i.e., latex particles, as the solid phase carrying the probe. The water suspensibility of the microparticles and their high surface area allow the kinetics of the hybridization reaction to be substantially as fast as the binding occurs when both nucleic acid strands are in solution. Moreover, the particulate nature of the solid phase enables rapid, convenient, and efficient separation of labeled material associated with formed hybrids away from the unreacted label.

The use of latex-immobilized probe is particularly advantageous in those hybridization formats involving detection by labeled anti-hybrid antibody reagent or by the use of means to label the sample nucleic acids prior to hybridization. In both cases, the measurement of label, particularly of the nonradioisotopic type, e.g., enzyme, can proceed very rapidly after separation of the latex-hybrid reaction product. Only one separation step is required, even where labeled anti-hybrid is used, since such reagent can be added to the hybridization mixture and resulting latex-hybrid:labeled antibody products separated by convenient means.

A particularly preferred embodiment of the present method uses enzyme-labeled anti-hybrid to detect hybridization products and acomplishes separation of the ultimate latex-hybrid:labeled antibody products by filtration. The filtered enzyme-labeled latex particles can then be readily measured visually or spectrophotometrically after applying a solution of a chromogenic or fluorogenic substrate for the enzyme.

The present invention is characterized by a number of significant advantages. Since DNA-latex forms a uniform suspension, it can be dispensed dropwise or by pipetting in the same manner as an aqueous liquid. Contact of the bulk liquid with the latex surface is optimal because the particles are dispersed throughout the liquid and the surface area is very large. Thus, diffusion paths for the soluble nucleic acid to the immobilized nucleic acid at the particle surface are short and rapid hybridization results.

After the hybridization step is completed and a labeled binding reagent is used to detect the hybrids, latex particles again provide a system favorable to rapid reaction because they are dispersed throughout the liquid and a large surface area is exposed to the binding reagent. When the binding is complete, excess binding reagent needs to be removed. This can be accomplished conveniently by collecting the particles on a filter. Wash solution can then be passed through the filter, flushing away excess labeled binding reagent. The wash can be completed in less than a minute.

Since the particles are concentrated into a small volume on the filter, signal from the label can be measured efficiently. For instance, if a colorimetric readout of an enzyme label is employed, the chromophore is formed in a small volume around the concentrated particles and can be measured more rapidly than if the chromogen were diluted into a volume of liquid. Assays employing latex particles can be completed in 30 minutes or less because the hybridization, washing and detection steps are rapid.

## BRIEF DESCRIPTION OF THE DRAWINGS

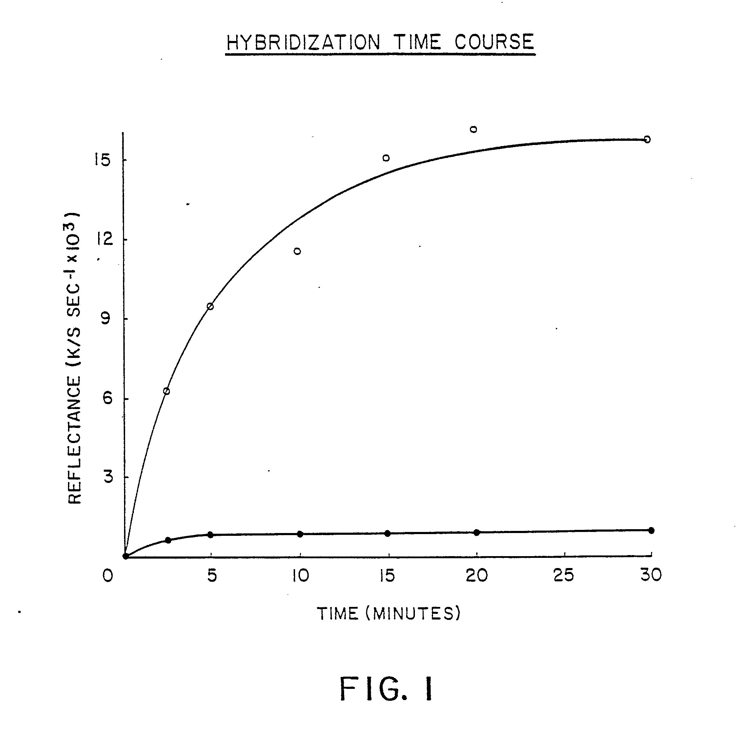

Fig. 1 is a graph showing the time course of the hybridization reaction between a sample nucleic acid in solution and latex-immobilized probe (open circles). The results with a buffer control (closed circles) are also shown. The data demonstrates that hybridization was effectively complete after only 15 minutes.

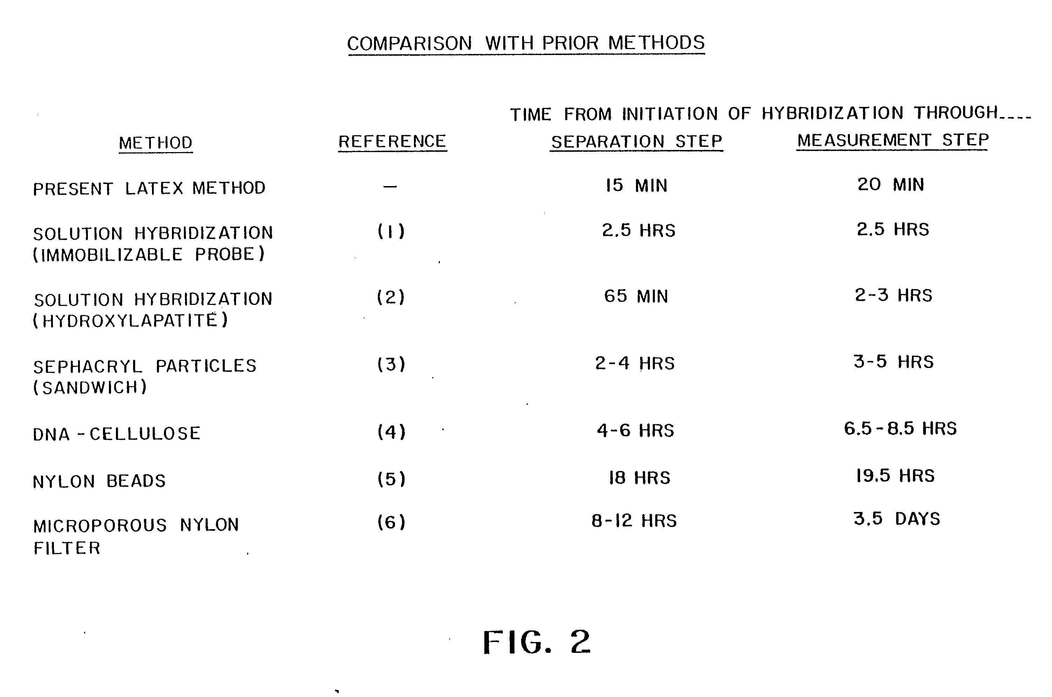

Fig. 2 is a table comparing the assay times for the present invention with those of the principal prior methods. For the present method, only 20 minutes are required from the time of hybridization initiation through the detection step, whereas the best times for the prior methods are in the range of 2-3 hours.

## DESCRIPTION OF THE PREFERRED EMBODIMENTS

The key feature of the present invention is the ability to conduct a hybridization assay from initiation of hybridization through at least the separation step in less than about 30 minutes, preferably less than 20 minutes, and optimally in about 15 minutes. Furthermore, the formats of hybridization that can be used in the present invention permit the use of rapid detection systems such that the complete assay, through the measurement of generated signal, can be completed in less than about 30 minutes, and usually in less than 20 minutes. These time periods are fundamentally independent of the concentration of the sequence of interest in the test sample, within the limits of detection of the signal generating system used, because large excesses of probe can be used to drive the kinetics and the hybridization reaction proceeds essentially as if both strands were in solution.

It should be recognized that latex microparticles, as the term is used herein, are, in general, discrete, water dispersable particles of such size and composition that they form a slowly settling suspension in the liquid hybridization medium. The particular chemical composition of such particles can vary as discussed hereinbelow.

In general, the probe can be any polynucleotide known in the art, or which can be generated by available methods, for the purpose of specific detection of the desired polynucleotide sequence by hybridization. The probe will be DNA or RNA and will comprise at least one single stranded base sequence substantially complementary to the sequence to be detected. Its length can vary widely, ranging from as few as about a dozen to as many as several thousand bases, and includes oligonucleotides having less than 50 bases.

Probes can be obtained in a variety of conventional manners. For example, in the case of RNA probes, RNA can be isolated as the natural products of cells, such as 5s, 16s and 23s ribosomal RNAs from bacterial or cellular transfer RNAs. It is also practical to isolate specific messenger RNAs from cells which specialize in production of large amounts of a protein for which the messenger codes.

In vitro synthesis of RNA probes can be accomplished with a vector which contains the very active Salmonella typhimurium bacteriophage SP6 transcription promoter. Vectors with multiple restriction endonuclease sites adjacent to the promoter are available. A DNA probe is cloned into the vector which is then propagated in a bacterial host. Multiple RNA copies of the cloned DNA probe can be synthesized in vitro using DNA dependent RNA polymerase from bacteriophage SP6.

DNA probes can be prepared from a variety of sources. An entire bacterial genome can be immobilized for a hybridization assay designed to detect bacteria in a typically sterile sample. The assay would be capable of detecting abundant bacterial RNAs such as ribosomal RNAs and transfer RNAs. Alternatively, specific DNA sequences can be cloned into well known plasmid or viral vectors and used as hybridization probes.

The samples to be assayed can be any material of interest, and will usually be of medical, veterinary, environmental or industrial significance. Human and animal specimens, body fluids and exudates, can be assayed by the present method, including urine, blood, milk, cerebrospinal fluid, sputum, fecal matter, lung aspirates, throat swabs, genital swabs and exudates, rectal swabs and nasopharangal aspirates. An assay of special interest is detection of bacteria in urine and cerebrospinal fluid. Where the test sample obtained from the patient or other source contains cells, the sample will be treated to release nucleic acids. If they are single stranded they can be assayed by the present method. If the nucleic acids are double stranded a procedure will be used to denature them. Denaturation is preferably accomplished by heating in boiling water or alkali treatment (e.g., 0.1 M sodium hydroxide), which, in some cases, can be used to simultaneously lyse cells. If the target material is an RNA, alkali denaturation will not be used because this condition degrades RNA. Also, release of nucleic acids can be obtained by mechanical disruption (freeze/thaw, abrasion, sonication), physical/chemical disruption (detergents such as Triton, Tween, sodium dodecylsulfate, osmotic shock or heat), or enzymic lysis (lysozyme, proteinase K, pepsin). These procedures will give a test medium with nucleic acids in single stranded form which can be assayed according to the present hybridization method.

The microparticles of the present invention will be of such composition that they are water suspensible and stable under hybridization conditions, and preferably will possess surface chemical groups enabling covalent linkage of nucleic acid probes. For convenience, they will preferably be of sufficient size to allow easy filtration, if such means of separation is used, or will be magnetic or magnetizable to allow magnetic separation.

For purposes of filtration, polystyrene latex particles of approximately 1 μm diameter and possessing either carboxyl or amido surface groups are ideal for this purpose. Carboxylate- and amide-modified latex are synthesized by the method of Mani (U.S. Pat. No. 4,094,841). More generally, latexes can be prepared by emulsion polymerization and suspension polymerization methods [Bangs, L.B. (1984) Uniform Latex Particles, Seragen Diagnostics Inc., Indianapolis, IN, USA]. Swollen emulsion polymerization can also be used [Ugelstad, J. et al (1980) Adv. Colloid and Interface Sci., 13:101-140].

Latex particles are often composed of polystyrene or cross-linked polystyrene which has a density of around 1.05 g/mL. Particles with a density of as low as 1.00 g/mL can be synthesized with a copolymer of vinyl-toluene/tertiary butylstyrene. Particles with very high density (up to 1.19 g/mL) can be prepared with polymethyl methacrylate. Other polymers and copolymers can be used to make particles. The best choice will depend on the density of the hybridization medium and the chemistry used for coupling DNA or RNA probes to the particle surface.

The most critical feature of the microparticles used to immobilize the probe is water suspensibility since this characteristic is principally responsible for the fast hybridization kinetics observed. As used herein, the property of water suspensibility will be understood to mean that substantially all, or at least a significant portion, of the particles originally put into suspension will remain generally uniformly distributed in the liquid and not settle out or float to the top of the hybridization solution during the time of the desired reaction. Thus, the outer limits on the rate at which the microparticles come out of suspension that can be tolerated will vary somewhat depending on the volume of the hybridization solution (more to the point, the distance from the surface of the solution down to the container bottom) and the time of hybridization. Given normal reaction volumes and a desired hybridization time of under 30 minutes, a useful definition of water suspensibility is that the turbidity of the suspension in the hybridization mixture will not decrease as much as about seventy percent (70%), more usually fifty percent (50%), and preferably twenty five percent (25%), in less than about 30 minutes.

Latex microparticles come out of suspension as a function of several parameters, including particle size, surface charge, and density, and the viscosity, density, and temperature of the liquid in which they are suspended. With regard to size, particles that are very small will be more difficult to separate from the hybridization medium in subsequent steps of the assay. On the other hand, very large particles will lack sufficient surface area for the immobilized nucleic acid probe and are less likely to remain suspended during the hybridization.

To achieve optimal hybridization conditions it is important that the particles remain as uniformly dispersed throughout the hybridization medium as possible. This minimizes the diffusion path between the soluble nucleic acid and the immobilized probe.

Given normal hybridization conditions in the liquid mixture, useful particles will generally be between about 0.1 and about 20 microns (μm), more usually between about 0.5 and about 5 microns in diameter, but may be as small as 0.05 microns or as large as 50 microns. The density of the particles will generally vary from between about 1.0 gram/mL to about 1.2 g/mL, with particularly preferred densities lying between about 1.03 g/mL and about 1.08 g/mL. Porosity is generally a noncritical feature of the particles. Large sized pores that allow access to macromolecules such as nucleic acids may increase the surface area of the particles and thereby allow a greater quantity of attached nucleic acid per weight of the particle. However, the pores may also decrease the efficiency of washing the particles.

The composition of the particles may be such that they contain surface groups which allow immobilization of nucleic acids. Examples of such groups are carboxylate, amide, epoxide, and phosphate; usually these functional groups are introduced onto the latex at the last stage of particle formation and they may be used directly or through further chemical modification.

It is also possible to coat particles of paramagnetic minerals such as magnetite ($Fe_3O_4$) with the polymers discussed above. These particles will be attracted to a magnet which will facilitate separation of the latex from liquid assay media. Paramagnetic minerals can also be coated with organosilanes which can react with the mineral surface and/or polymerize on the surface to give water suspensible particles with diameters of about 0.1 to 1.5 μm diameters [Whitehead, R.A. et al (1985) U.S. Pat. No. 4,554,088]. The organosilanes can have functional groups which can be used subsequently for coupling of DNA or RNA probes.

DNA or RNA probes can be attached to latex particles by adsorption through electrostatic and/or hydrophobic interactions, however, covalent attachment is preferred because the probe is unlikely to dissociate from the latex during storage. A wide variety of covalent coupling techniques can be employed. A preferred method involves introduction of diazonium groups onto the latex and subsequent reaction with the guanine, thymine, and uracil residues of the polynucleotide [Noyes, B.E. and Stark, G.R., (1975) Cell 5:301-310; Reiser, J., et al, (1978) Biochem. Biophys. Res. Commun. 85:1104-1112]. Phosphate ester groups can be introduced onto the latex and coupled to the probe through activation with a carbodiimide [Bautz, E.K.F. and Hall, B.D. (1962) Proc. Nat'l. Acad. Sci. USA 48:400-408; Adler, A.J. and Rich, A. (1962) J. Am. Chem. Soc., 84:3977-3979]. Hydroxyl groups on the latex can be used with coupling through phosphodiester links formed between the terminal phosphate of the polynucleotide and the hydroxyls by water soluble carbodiimide activation [Rickwood, D. (1972) Biochim. Biophys. Acta 269:47-50; Gilham, P.T. (1968) Biochem. 7:2809-2813] or by coupling nucleophilic sites on the polynucleotide with cyanogen bromide activated hydroxyls [Arndt-Jorin, E.J., et al, (1975) Eur. J. Biochem. 54:411-418; Linberg, U. and Eriksson, S. (1971) Eur. J. Biochem. 18:474-479]. Further, the 3'-hydroxyl terminus of RNA probes can be oxidized with periodate and coupled by Schiff base formation with latex bearing amine or hydrazide groups [Gilham, P.T. (1971) Meth. Enzymol. 21:191-197; Hanoske, H.D., et al, (1979) Meth. Enzymol. 49:172-181]. Latexes with nucleophilic sites can be reacted with cyanuric chloride and then with the polynucleotide [Hunger, H.D. et al (1981) Biochim. Biophys. Acta 653:344-349]. In addition, photoactivatable groups can be introduced onto the latex and DNA or RNA probes can be coupled by activation with light [Dattagupta, N. (1985) U.S. Pat. No. 4,542,102].

An especially preferred latex-probe conjugate is DNA or RNA covalently coupled to carboxylate-modified latex (CML). A linker, N-[17-amino-3,6,9,12,15-pentaoxaheptadecyl]-3-N-BOC-aminophenylacetamide (amino-PEG-BOC-aminophenylacetamide), is coupled to CML with water soluble carbodiimide. Then the BOC blocking group is removed with acid and the aryl amine diazotized and coupled to the probe DNA or RNA. Alternatively, N-[17-amino-3,6,9,12,15-pentaoxaheptadecyl]-3-nitrobenzoamide (amino-PEG-nitrophenylacetamide) is coupled to the carboxyl groups of CML with a water-soluble carbodiimide. The nitro group is reduced and converted to the diazonium ion which couples to the probe nucleic acid.

There are principally two hybridization formats which can take unique advantage of the use of latex-immobilized probe according to the present invention. The first is particularly preferred and can be conveniently referred to as the anti-hybrid format, and the second is known as the sample labeling format. Both of these have been essentially described in the literature (European Patent Publication 163,220 and European Patent Application No. 87 102 577.1, filed February 24, 1987, supra) but will be briefly reviewed here.

In the anti-hybrid format, hybrids formed between the sample sequence of interest and the immobilized probe are detected by binding of a reagent that selectively binds such hybrids, but does not bind substantially to single stranded nucleic acids. Immobilized hybrids to which the hybrid-directed reagent has bound are separated from unbound reagent and the presence of the reagent detected.

The hybrid-directed reagent will conveniently be an antibody or fragment thereof selective for the hybrids, and can particularly be selected from (a) antibody to DNA RNA or RNA RNA hybrids, where one of the probe and the sequence to be determined is DNA and the other is RNA, or where both are RNA, respectively [see European Patent Publication 163,220]; (b) antibody to intercalated hybrids, where the hybrids that result between the probe and the sequence to be determined are formed to comprise a nucleic acid intercalator bound thereto as an intercalation complex [see U.S. Pat. No. 4,563,417]; and (c) antibody to double-stranded DNA, where both the probe and sample sequence are DNA [see U.S. Pat. No. 4,623,627]. Preferably the anti-hybrid reagent will be labeled with a detectable chemical group for ready detection. While essentially any of the known labels can be used, particularly useful labels are enzymatically active groups, e.g., enzymes, substrates and coenzymes, fluorescers, chromophores, luminescers, metal sols, or ligands, e.g., biotin or haptens, that can be detected by binding of a binding partner labeled with any of the aforementioned particularly useful labels. Such labels are capable of rapid and convenient generation of a detectable signal, particularly an optical signal, that allows the entire assay procedure from initiation of hybridization through separation and detection to be completed in less than about 30 minutes.

The sample labeling format involves a preliminary step of treating the test sample to chemically modify the nucleic acids therein to introduce a detectable label. Those labeled nucleic acids that hybridize to the immobilized probe will contain the sequence of interest and, after separation of unhybridized labeled nucleic acids, can be detected directly. Preferred labels are those described above. The label can be introduced by any convenient method that is available or that can be devised, however, particularly preferred is the use of labeled, reactive, nucleic acid-binding ligands, particularly those that are photoreactive. Photoreactive intercalation compounds that have been conjugated to a label are particularly useful and are described in the literature (European Patent Publication 131,830; European Patent Application No. 87 102 577.1, supra; and Forster et al (1985) Nucl. Acids Res. 13:745).

The separation of the latex particles from the hybridization mixture required to complete the assay can be accomplished in any convenient manner, usually by filtration, centrifugation, or magnetic attraction. A wide variety of filtration media are available and the porosity of a filter will be an important consideration. If small diameter latexes are employed, a small pore diameter will be required. Depth filters such as glass fiber filters are useful because they retain latexes efficiently even when the nominal pore size is substantially larger than the mean latex particle diameter. Such filters are attractive because they pass liquids at acceptable flow rates. Flow can be facilitated by placing a vacuum under the filter. It is often convenient to place the filter on an adsorbant that soaks up the waste liquids. This type of system usually gives rapid flow rates without a vacuum (see for example the devices described in U.S. Pat. Nos. 3,811,840; 4,246,339; 4,366,241; and 4,632,901). Readout of the assay signal can be quantitated with an instrument or can be visually observed for color especially if a yes/no result is satisfactory.

The present invention will now be illustrated, but is not intended to be limited, by the following examples.

## EXAMPLES

Probe DNA covalently immobilized on latex particles was hybridized with ribosomal RNA (rRNA) from Escherichia coli and hybrids were detected colorimetrically with enzyme-labeled antibody specific for DNA:RNA hybrids. Carboxymethyl latex (CML) was coupled to amino-PEG-BOC-aminophenylacetamide. Then the aniline group was deblocked and diazotized. The diazonium ion reacted efficiently with probe DNA to give up to 7 μg DNA/mg latex.

The DNA-latex was hybridized with rRNA and then β-galactosidase-labeled antibody to DNA:RNA was allowed to bind to the hybrids. Excess β-galactosidase anti-DNA:RNA conjugate was washed away by collecting the latex particles on a specially designed glass fiber filter strip. The amount of β-galactosidase-anti-DNA:RNA conjugate bound to the hybrids was measured by placing the filter strip on a Seralyzer® reflectance instrument (manufactured by Ames Division, Miles Laboratories, Inc., Elkhart, IN, USA), adding a chromogenic β-galactosidase substrate, and measuring the rate of color formation.

1. Molecular Cloning of E. coli and B. subtilis rRNA Genes

DNA probes were prepared by cloning restriction fragments containing the 23S rRNA gene from E. coli and B. subtilis into M13mp18 and M13mp19 [Messing et al, (1977) Proc. Nat'l. Acad. Sci., 75:3642] A 3.2 Kb DNA fragment containing the E. coli 23S rRNA gene was obtained from pN01301 [Jinks-Robertson et al (1983) Cell 33:865] by digestion with restriction endonucleases XbaI and XmmI. These sites were chosen after computer analysis of the DNA sequence of the rrnB operon showed that they would give a fragment with the entire 2904 base pair 23S rRNA gene sequence without the adjacent 5S rRNA gene. The fragment was cloned into the M13 vectors which had been previously digested with endonucleases XbaI and SmaI.

A 2.0 Kb DNA fragment containing two-thirds of the B. subtilis 23S rRNA gene from the rrnB operon was obtained from p14B1 by digestion with restriction endonucleases BamHI and SmaI. This fragment contained all of the 23S rRNA gene except 408 and 459 base pairs coding for the 5' and 3' terminal sequences, respectively [Green, et al (1985) Gene, 37:261]. This was cloned into XbaI and SmaI restriction sites on the M13mp18 vector. Virion DNA from these clones, designated M13-23SE and M13-23SB, respectively, was used as the probe in subsequent hybridization experiments. Recombinant DNA molecules were constructed in vitro by ligation, with T4 DNA ligase, of restriction endonuclease-linearized M13mp18 and M13mp19 replicative form DNAs to restriction endonuclease-generated DNA fragments of plasmids containing the 23S rRNA genes from E. coli and B. subtilis at 12°C for 16 to 18 hours with the buffer described by Maniatis et al, (1982) Molecular Cloning, A Laboratory Manual, Cold Spring, Harbor, NY. Successful ligation was monitored by subjecting reaction samples to electrophoresis on 1% agarose gels along with DNA molecular size markers. Competent E. coli were transformed by the ligation reaction products and appropriate dilutions were plated to detect recombinant phage with soft agar overlay medium containing isopropyl-β-D-thiogalactopyronoside and 5-bromo-4-chloroindolyl-β-D-galactopyranoside [Messing, (1983) Meth. Enzymol., 110:20].

Recombinant phage containing the appropriate cloned 23S rRNA gene fragment were identified by screening the recombinant phage plaques for RF DNA with appropriately sized DNA inserts. Individual plaques were dug from the soft agar overlay and suspended in 1 mL of 0.1 M NaCl, 8 mM MgSO₄, 50 mM Tris-HCl, pH 7.5, 0.01% gelatin for 1 hour which was then used to infect 5 mL cultures of E. coli TG1 in tryptose yeast extract peptone medium. After 3-4 hours of vigorous aeration at 37°C, the cultures were collected by centrifugation at 3000 x g for 10 minutes. The cell pellets were suspended in 1.6 mL of buffer consisting of 8% (w/v) sucrose, 5% Triton X-100, 50 mM EDTA, 50 mM Tris-HCl (pH 8.0). The suspensions were transferred to Eppendorf centrifuge tubes containing 50 μL of lysozyme (10 mg/mL in water) and incubated at 95°C for two minutes. The resulting cell lysates were subjected to centrifugation at 13,000 x g for five minutes and 0.7 mL samples were removed to a clean tube. Cold isopropanol (0.7 mL) was added and the tubes were placed at -18°C for ten minutes. The tubes were centrifuged at 13,000 x g for five minutes in the cold, the supernatants were discarded and the DNA precipitates were dried briefly in vacuo. The precipitates were dissolved in 50 μL of water and samples were electrophoresed on 0.7% agarose gels to establish the presence of vector RF DNA of increased size. Appropriate samples were then subjected to restriction enzyme digestion and gel electrophoresis to establish the presence of the 23S rRNA gene fragments.

## 2. Synthesis of Amino-PEG-BOC-aminophenylacetamide

### 3-Aminophenylacetic Acid.

A solution containing 10 g of 3-nitrophenylacetic acid (55 mmol) and 300 mg of 10% Pd/C catalyst in 125 mL of ethanol was hydrogenated on a Paar apparatus at 50 psi of H₂ for three hours. The mixture was then filtered through a Celite filter and the cake was washed with ethanol. The combined filtrate was concentrated in vacuo to give 7.18 g of a white powder which was used without further purification (86% yield). mp. 131-132°.

An analytical sample was prepared by recrystallization from water. mp. 145-8° (Lit 148-9°).
$^1$H NMR (60 MHz, CDCl₃) δ: 3.6 (s, 2H); 7.0-7.6 (m, 4H).
IR (KBr) cm⁻¹: 3000, 1592, 1377 cm⁻.
Mass Spectrum (EI) m/e:
 151.1 (M⁺, 54.5%);
 152 (M⁺1, 5.3%);
 106 (M⁺-45, 100%).

### 3-N-BOC-Aminophenylacetic Acid.

A solution containing 3.02 g of 3-aminophenylacetic acid (20 mmol) and 6.97 mL of diisopropylethylamine (5.17 g, 40 mmol) in 40 mL of CH₂Cl₂ was added to a solution containing 4.65 g of di-tert-butyl carbonate (21.3 mmol) in 20 mL of CH₂Cl₂. After three hours, 13.94 mL of diisopropylethylamine (80 mmol) and 9.16 g of di-tert-butyl carbonate (42 mmol) were added, resulting in a complete disappearance of starting material (as noted by TLC) after three hours. The solvents were then evaporated in vacuo and the reaction mixture flash chromatographed on 500 g of SiO₂-60 eluted with 19:1 CHCl₃-CH₃OH solvent mixture. Fractions of 25 mL were taken. Fractions of pure product (54-85) were pooled and concentrated to give 1.622 g of a white solid on which the following analytical data was obtained (32% yield). mp 102-104°.
Analysis: Calculated for C₁₃H₁₇NO₄:
 C, 62.14; H, 6.82; N, 5.56
Found:
 C, 62.19; H, 6.78; N, 5.50
IR (CHCl₃) cm⁻¹: 3400, 3010, 3000. 2940, 1725, 1620, 1600, 1529, 1240, 1159.
NMR (60 MHz, CDCl₃) δ: 1.5 (s, 9H); 3.6 (s, 2H), 6.95 (m, 2H, ArH and NH); 7.3 (3H, Ar-H); 9.67 (m, CO₂H).
Mass Spectrum (EI) m/e: 251.2 (M⁺, 2.8%); 252.2 (M+1, 0.4%).

Fractions 86-180 contained impure product which were then pooled, concentrated, and rechromatographed on a 200 g column of SiO₂-60 to yield an additional 891 mg of product. mp 103-105°.
Analysis: Found: C, 61.35; H, 6.68; N, 5.50.

The combined yield of product was thus 2.513 g (50% yield).

### N-(17-Amino-3,6,9,12,15-Pentaoxaheptadecyl-3-N-BOC-Aminophenylacetamide.

To a solution containing 1.265 g of N-hydroxysuccinimide (11 mmol) and 2.51 g of 3-N-BOC-aminophenylacetic acid (10 mmol) in 20 mL of $CH_2Cl_2$ at 0° was added dropwise over a five minute period of solution containing 2.166 g of dicyclohexylcarbodiimide (DCC) in 20 mL of $CH_2Cl_2$. The resulting cloudy solution was allowed to stir at 0° C for 0.5 hour and was then allowed to warm to ambient temperature over a four hour period. The mixture was then filtered and the precipitate washed twice with 5 mL portions of $CH_2Cl_2$. The combined filtrates were then placed in an addition funnel and added dropwise to a solution containing 7 g of 2,17-diamino-3,6,9,12,15-pentaoxaheptadecane (25 mmol) [Kern, et al (1979) Makromal. Chem., 180:2539] in 50 mL of $CH_2Cl_2$. The resulting mixture was allowed to stir overnight at ambient temperature. The resulting mixture was then concentrated in vacuo and flash chromatographed on a 500 g SiO₂-60 column eluted with a 90:10:1 CHCl₃-CH₂OH-conc. NH₄OH solvent mixture. Fractions of 25 mL were collected. Fractions 66-81 were pooled and concentrated to give 1.77 g of product as a light yellow oil (31% yield).

Analysis: Calculated for $C_{25}H_{43}N_3O_8$ 1/2 $H_2O$:

   C, 57.45; H, 8.29; N, 8.04

Found:

   C, 57.10; H, 8.15; N, 8.16

$^1$H NMR (60 MHz, CDCl₃) δ: 1.5 (s, 9H); 2.1 (m, NH₂); 3.4-3.7 (m, 26H); 6.6 (m, NH); (m, NH); 7.0 (m, NH); 7.3 (m, H, ArH).

IR (CHCl₃) cm⁻¹: 3009, 2900, 1729, 1660, 1527, 1235, 1215, 1158, 1099.

Mass Spectrum (FAB) m/e: 514 (M + 1, 28.8%);

### 3. Preparation of Diazonium-Latex

Diazonium-latex was prepared by derivatizing carboxylate-modified latex (CML, Seragen Diagnostics, Indianapolis, IN, USA), with amino-PEG-BOC-aminophenylacetamide, deblocking the aniline group, and diazotizing the amine with nitrous acid. (CML is sold as a 10% (s/v) suspension of 1.250 ± 0.015 μm particles containing ~ 50 nmole carboxylate groups per mg latex.)

For a medium sized preparation, 1 mL of 10% CML was filtered on hydrophilic Durapore (polyvinylidenedifluoride) membrane filters (Millipore Corp., Bedford, MA, USA) having a pore size of 0.45 μm and rinsed with deionized H₂O. After drying slightly in vacuo over anhydrous CaSO₄, the resulting latex cake was easily scraped from the membrane filter, resuspended into ~ 1 mL deionized H₂O (sonication, vortexing) and placed in a test tube. To this was added ~ 1 g of moist AG 501/X8 mixed bed ion-exchange resin (20-50 mesh) (Bio-Rad Laboratories, Richmond, CA USA) to remove all electrolytes, anionic surfactants, and water-soluble polymeric material. The latex was separated from the resin using a scintered glass filter, rinsing the resin with deionized water, and collecting by filtration as described above.

The latex was resuspended in 0.1 M pyridine HCl buffer, pH 4.5, containing ~25 mg (50 μmole) of amino-PEG-BOC-aminophenylacetamide. To this was added ~ 95 mg (0.5 mmole) of 1-ethyl-3-(3-dimethylaminopropyl)carbodiimide hydrochloride in two equal portions, the second part one hour after the first. After mixing the reaction suspension at room temperature a total of at least four hours, the latex was again collected by filtration and rinsed with deionized water.

The latex was resuspended in ~1 mL of water and an equal volume of concentrated HCl added. The mixture was gently shaken for 15 minutes at 80°C before cooling on ice and then diluting with 3 mL of 0.2 M NaNO₂. After 5 minutes, the diazonium latex was collected on a filter and quickly rinsed with cold 0.1 M sodium acetate buffer, pH 4.2. The latex cake was allowed to dry in vacuo for only 3 minutes, since the diazonium groups are labile.

The latex was resuspended in 0.1 M sodium acetate buffer, pH 4.2, to a final volume of about one mL. A 2.0 μL sample of the diazonium-latex was diluted five-fold with buffer and assayed for diazonium groups using the assay method described below. Two 40 μL samples of the diazonium-latex were pipetted onto tared pre-dried cover slips and immediately weighed. The samples were dried for at least four hours in vacuo (oil pump) and at 55°C. The dried samples were again weighed. The weight of the dry diazonium-latex (dried sample minus cover slip tare minus buffer salt contribution - ~70 μg per sample) divided by the weight of the liquid (pre-dried sample minus cover slip tare) gave the solids content of the suspension.

### 4. Assay for Diazonium Groups

Diazonium groups were quantitatively determined using 1-naphthol-8-amino-5,7-disulfonic acid in a back-titration assay method. After resuspending diazotized particles in 0.1 M sodium acetate buffer, pH 4.2, a 50 μL sample (or dilution) was mixed at room temperature with 50 μL 2 mM 1-

naphthol-8-amino-5,7-disulfonic acid in 10 mM Tris-HCl, pH 8.0. The latex particles quickly turned lavender if diazonium groups were present. After five minutes, the samples were diluted with 0.9 mL deionized water and the particles sedimented by centrifugation at 13,000 x g for 2 minutes. The absorbances of the blank (no naphthol added), standard (no latex diazonium added), and samples of immobilized diazonium groups were determined at 340 nm. The difference in signal between the latex samples and the standard (minus background) was used to calculate the amount of naphthol removed from solution by the immobilized diazonium groups.

## 5. Preparation of DNA-Latex

The DNA-latex was prepared by adding a mixture of M13-23SE and M13-23SB probe DNA to freshly prepared diazonium-latex at a ratio of 10 μg DNA per 1 mg latex. The remaining diazonium-latex, from the preparation described in section 3 above, was mixed with 151 μL of 1.6 mg/mL M13-23SE and 433 μL of 0.52 mg/mL M13-23SB. The mixture was agitated overnight at ~ 5°C, then warmed to room temperature for an additional four hours. The DNA-latex preparation was diluted with ~ 3 mL 1 x SSC (0.015M sodium citrate buffer, pH 7.0, 0.15M NaCl), 0.1% sodium dodecylsulfate (SDS), filtered, and rinsed with the same solution. After a final rinse with 10 mM Tris HCL, pH 7.4, 1 mM ethylenediaminetetraacetic acid (EDTA), the latex cake was dried in vacuo over CaSO₄.

The DNA-latex was resuspended in 10 mM Tris-HCl, pH 7.4, 1 mM EDTA to a final volume of 0.9 mL to make a 100 mg/mL latex suspension. Two samples of 2.5 and 5 μL were diluted to 50 μL with buffer and 50 μL of 2 x hybridization solution, minus salmon sperm DNA (see below), was added. The samples were pre-hybridized at 80°C for 30 minutes, diluted with deionized water to ~ 1.5 mL and centrifuged to pellet the latex. After decanting the supernatant, the pellets were resuspended in 100 μL of buffer and the DNA quantitated using the fluorescent assay described below. DNA-latex preparations routinely contained 2.5 to 7.0 μg DNA per mg latex.

To the remaining DNA latex preparation was added one volume of 2 x hybridization solution [8 x SSPE, 4% sodium polyacrylate, 0.2% SDS, 0.2 mg/mL denatured alkali-treated salmon sperm DNA (Boguslawski et al (1986) J. Immunol. Meth. 89:123)] (1 x SSPE is 10 mM sodium phosphate buffer, pH 7.4, 0.15M NaCl and 1.0 mM EDTA) and the mixture prehybridized at 80°C for 30 minutes. The mixture was diluted 10-fold with water and filtered, rinsing with 2 x SSPE. The DNA-latex cake was resuspended in 2 x hybridization solution to a concentration of 4 μg/mL DNA. Efficient resuspension to fine particles was achieved best by first sonicating the latex in a portion of water. After resuspension in the water, the other components of the hybridization solution were added as concentrated solutions (0.4 volume of 20 x SSPE, 0.4 volume of 10% SPA, 0.01 volume of 20% SDS, 0.067 volume of 3 mg/mL denatured alkali-treated salmon sperm DNA).

## 6. Fluorescent Assay for DNA on Latex

Quantitation of probe DNA immobilized on latex particles was performed using a modified method by Hinegardner (1971) Anal. Biochem. 39:197. The method involves a reaction of 3,5-diaminobenzoic acid with the deoxyribose of the DNA to form a fluorescent product. After the reaction, the latex particles were diluted with 1 mL of 1 M HCl and pelleted by centrifugation (two minutes at 13,000 x g). The fluorescence was determined after mixing 1 mL from the sample with an additional 1 mL of 1 M HCl. The assay was sensitive to 1 μg/mL (0.1 μg/assay) with very good precision.

## 7. Conjugation of β-D-Galactosidase and the Fab' Fragment of DNA RNA Antibody

Mouse monoclonal IgG to DNA:RNA hybrid was prepared as described previously [Boguslawski, et al, supra]. F(ab')₂ fragments were obtained by digestion with a 1:33 weight ratio of pepsin to IgG for 16 hours at 37°C in 0.1 M sodium acetate, pH 4.2. The digestion products were chromatographed on a Sephacryl S-200 column (Pharmacia, Inc., Piscataway, NJ, USA) in 10 mM sodium phosphate buffer, pH 7.0, 0.15 M NaCl.

A portion of the F(ab')₂ was labeled with dichlorotriazinylaminofluorescein (DTAF) to be used as an antibody tracer in conjugate preparation. The labeling reaction was carried out for 1 hour in 0.1 M sodium borate buffer, pH 9.0, with 3:1 molar ratio of DTAF to F(ab')₂ [Blakeslee and Baines (1976) J. Immunol. Meth., 13:305]. The labeled antibody was separated from free DTAF on a BioGel P6-DG (Bio-Rad Laboratories) column. The molar DTAF:F(ab')₂ ratio calculated from an empirically derived formula was 2.1 [The and Feltkamp (1970) Immunol., 18:865].

F(ab')₂ was mixed with DTAF-F(ab')₂ in 20:1 ratio and reduced to Fab' in 0.1 M sodium phosphate buffer, pH 7.0, 0.15 M NaCl, 1 mM EDTA, 10 mM dithiothreitol. The reduction was carried out for 3 hours at room temperature. The Fab' fragments were isolated on a BioGel P6-DG column in 0.1 M sodium phosphate, pH 7.0, 0.15 M NaCl, 1 mM EDTA and used immediately for coupling to maleimido-β-D-galactosidase prepared as described below. The sulfhydryl content of Fab' as determined by the Ellman method [Habeeb (1972) Meth. Enzymol., 25:457] was 2.5-3.0 moles sulfhydryl per mole of Fab'.

8. Synthesis of 1,17-Dimaleimido-3,6,9,12,15-pentaoxaheptadecane (BMP)

A solution containing 2.80 g of 1,17-diamino-3,6,9,12-15-pentaoxaheptadecane (10 mmol) in 20 mL of dry tetrahydrofuran (THF) was added dropwise over 1 hour to a stirred solution containing 4.50 g of maleic anhydride (45 mmol) in 20 mL of THF. After 1 hour, the reaction mixture was filtered and the filtrate concentrated to an oil in vacuo at 50°C to give 6.34 g of the bis-maleic acid intermediate as a crude yellow paste. Hydroxybenzotriazole hydrate (2.97 g, 22 mmol) was then added and the residue was thrice azotropically distilled in vacuo with 20 mL aliquots of DMF. The residual oil was then placed under an argon atmostphere, dissolved in 20 mL of DMF, cooled to 0°C, and treated with 4.54 g (22 mmol) of dicyclohexylcarbodiimide. The resulting mixture was allowed to stir for 1 hour at 0°C and then overnight at ambient temperature. The resulting dark brown mixture was filtered and concentrated to give 5.62 g of a crude, dark brown oil. The sample was purified by flash chromatography on SiO₂-60 (230-400 mesh) using a 1% CH₃OH-CHCl₃ solvent mixture. Fractions containing the pure product were pooled and concentrated to give 1.62 g of an oil (37% yield).

Analysis: Calc'd for C₂₀H₂₈N₂O₉:

C, 54.53; H, 6.41; N, 6.36

Found:

C, 54.96; H, 6.28; N, 6.48

PMR (60 MHz) CDCl₃Δ: 3.63(s, 10H); 3,70 (s, 14H); 6.70 (s, 4H).

IR (CHCl₃) cm⁻¹: 2860, 1710, 1405, 1100 cm⁻¹.

Mass Spectrum (FAB) m.e: 441 (M + 1, 51%).

9. Preparation of the Maleimide-β-D-Galactosidase

β-Galactosidase was prepared by the method of Fowler and Zabin (1983) J. Biol. Chem., 258:14354 and stored as a 50% ammonium sulfate suspension. An aliquot of enzyme suspension was centrifuged and the pellet was dissolved in 0.1 M sodium phosphate buffer, pH 7.0, 0.15 M NaCl. Dithiothreitol was added to a final concentration of 2 mM, the mixture was incubated for 4 hours at 25°C, and then chromatographed on a BioGel P6-DG column in 0.1 M sodium phosphate, pH 7.0, 0.15 M NaCl, 1 mM EDTA. The sulfhydryl content was 9.1 to 10.4 moles per mole of enzyme. Next, reduced β-galactosidase was reacted for 1 hour at room temperature with a 200-fold molar excess of BMP (section 8 above), freshly prepared in 0.1 M sodium phosphate buffer, pH 7.0, 0.15 M NaCl, 1 mM EDTA. The mixture was chromatographed on BioGel P6-DG in the same buffer and the activated β-galactosidase was used immediately for coupling with Fab'. The maleimide content of activated β-galactosidase was determined by reaction of a portion of the derivatized enzyme with excess glutathione and then measuring the excess glutathione with Ellman's reagent [Habeeb (1972), supra]. The maleimide content was 6.9 to 10.5 moles per mole of enzyme.

10. Preparation of the Fab'-β-Galactosidase Conjugate

Maleimido-β-galactosidase was combined with Fab' in a 1:5 molar ratio. The final concentration of maleimido-β-galactosidase was 1.5 μM. The conjugation reaction was carried out for 22 hours at 5°C with stirring. Some aggregated material formed and was removed by centrifugation. The supernatant was chromatographed on a 1.5 x 46 cm BioGel A-1.5m (Bio-Rad Laboratories) column in 10 mM sodium phosphate buffer, pH 7.0, 0.15 M NaCl. The fractions were examined for enzyme activity, for absorbance at 280 nm, and for fluorescence of DTAF-Fab' using 492 nm excitation and 512 nm emission. The fractions showing enzyme activity and fluorescence contained conjugate and they were pooled and stored at -15°C in 0.1 M sodium phosphate, pH 7.0, 0.15 M NaCl, 0.1% NaN₃, 1 mg/mL bovine serum albumin (BSA) containing 50% glycerol. Based on the recovery of enzyme activity and fluorescence, the conjugate contained 4.1 moles Fab' per mole of enzyme.

## 11. Preparation of Fab'-β-Galactosidase Solutions

The conjugate was diluted to a working concentration of ~ 40 μg/mL in 50 mM sodium phosphate buffer, pH 7.4, 10 mM MgCl₂, 0.15 M NaCl, 0.5% BSA, 0.5% Tween 20, 0.05% NaN₃ and filtered through a GF·F glass fiber filter (Whatman Ltd., Maidstone, England), then a 0.22 μm filter (Millipore Corp., Bedford, MA, USA) to remove aggregated conjugate.

## 12. Fabrication of Filter Strips

Ten centimeter wide 31ET Whatman paper was impregnated with 0.2% ethylcellulose in toluene, dried and cut into 15 cm cards. A 10 mm wide strip of porous adhesive tape (Fasson, Painesville, OH, USA) was affixed to the impregnated 31 ET cards (5 mm from one edge) and 12 mm wide GF·F glass fiber ribbons were laminated on top of the adhesive tape. The cards were cut into strips.

## 13. Vacuum Filter Device

A vacuum filtration device was constructed to permit insertion of the GF·F filter end of the strip up to a stop. When the strip was inserted, the GF·F filter was positioned over a vacuum port; as the device handle was lowered to the down position, a beveled reagent load port was lowered to seat itself tightly over the edges of the GF·F filter pad (12 mm x 5 mm) and a vacuum line was simultaneously engaged. Assay reagents were pipetted into the reagent port (vacuum on) and filtered through the GF·F pad and support backing. Liquid was collected in a trap for easy disposal. When all reagents are filtered, the strip was removed from the vacuum device and placed onto the table of a Seralyzer® instrument for addition of substrate.

## 14. E. coli Lysate Preparation

E. coli lysates were prepared from cultures of E. coli in logarithmic growth phase [4-7 x 10⁸ colony forming units (CFU)/mL]. Cells, sedimented from culture medium, were resuspended in lysis medium (50 mM Tris buffer (pH 7.3), 10 mM EDTA, 200 μg/mL lysozyme) and incubated at 37°C for 10 minutes. Cellular debris was removed by centrifugation, the supernatant was made 0.1% SDS and the volume was adjusted to the original culture volume with 50 mM Tris buffer, pH 7.3, 10 mM EDTA. Aliquots of lysates in 50 mM Tris-HCl, pH 8.0, 10 mM EDTA, 0.1% SDS were also incubated at 60°C for 10 minutes were stored at -20°C until dilution to desired working levels.

## 15. Preparation of rRNA

rRNA was prepared from E. coli cells and the 23S component was isolated by sucrose density gradient centrifugation [McConkey (1967) Meth. Enzymol., 12a:620 and Takanami (1967) Meth. Enzymol., 12a:491].

## 16. Hybridization of DNA-Latex with rRNA

The method consisted of the following steps:

(a) Fifty microliters of rRNA (1.0 ng/mL) or E. coli lysate was pipetted into a 1.5 mL conical centrifuge tube.

(b) Fifty microliters of 2x hybridization solution (supra) containing probe DNA-latex was added and mixed thoroughly.

(c) The tubes were incubated at 80°C for 10 minutes (or other indicated time).

(d) One hundred microliters of 2 μg/mL Fab'-β-galactosidase conjugate was added, mixed well and allowed to stand at room temperature for 30 minutes (or other indicated time).

(e) Then, 500 μL of wash buffer [50 mM sodium phosphate, pH 7.4, 0.5 M NaCl, 10 mM MgCl₂, 0.5% (v/v) Tween 20 and 0.05% (w/v) sodium azide] was added and the contents were decanted onto the filter pad of a strip mounted in the vacuum device with the vacuum on. The tube was washed with 500 μL of wash buffer which was decanted onto the filter. The filter was washed with 2.0 mL wash buffer.

(f) The filter strip was removed from the filtration device and placed on the Seralyzer® reflectance meter table. Thirty microliters of 5mM substrate reagent [50 mM sodium phosphate buffer, pH 7.4, 5mM MgCl₂ 0.05% sodium azide and 5.0 mM chlorophenol red-β-D-galactopyranoside, Kuhr (1985) German Offen. DE 3345748] was pipetted onto the filter. The reflectance at 570 nm was measured at 5 second intervals from 30 to 60 seconds. The reflectance values were converted to K/S values [Greyson (1981) J. Automat. Chem., 3:65] and the rate was calculated.

### 17. Hybridization Time Course

A series of hybridization mixtures were prepared with 50 μL of buffer or 50 μL of E. coli lysate at a level of 10,000 CFU/mL in each assay tube. The tubes were incubated in an 80°C water bath for given periods and then assayed for hybrids as described above using 30 minutes for binding of Fab'-β-galactosidase at 37°C. The assay responses are plotted in Fig. 1 versus the hybridization time. Results for mixtures with lysate and buffer controls are indicated by open and closed circles, respectively. The data establishes that the hybridization was complete in 15 minutes.

### 18. Total Assay Time

In a study directed to shortening the total assay time, the binding of Fab'-β-galactosidase conjugate to DNA:RNA in hybridization mixtures was examined in more detail. A series of hybridization mixtures were prepared with E. coli lysate equivalent to 2500 cells/assay. Following hybridization, 0.4 mL of 2.5 μg Fab'-β-galactosidase was added, mixed and allowed to stand for various periods. The conjugate binding was terminated by collecting the latex on glass fiber filters and washing with wash buffer adjusted to pH 7.9 instead of 7.4. The wash buffer also contained 1.0 mM 7-β-D-galactopyranosyloxy-9,9-dimethyl-acridin-2-one (synthesized as described in U.S. Patent Application Serial No. 939,855, filed December 9, 1986, and commonly assigned herewith). The rates of color formation measured by reflectance at 634 nm showed that the conjugate binding proceeded rapidly for the first two minutes and then proceeded at a much slower rate. A binding time of 3 minutes was chosen for the conjugate.

Further work employed 10 minutes for hybridization, 3 minutes for conjugate binding and 3 minutes for measurement of enzyme activity. The blue color produced by enzymic hydrolysis of the substrate could clearly be observed visually when the equivalent of 2500 E. coli cells were added to the hybridization mixture. Thus, this number of cells could be detected by a procedure that required less than 20 minutes.

### 19. Comparison with Prior Art Hybridization Methods

A study was conducted of the literature and prior methods used in the present laboratory in order to determine the time limitations of the currently known hybridization methods. The results of the study were used to construct the table shown in Fig. 2 of the drawings. The references are as follows:

1. Yehle et al. Accepted for publication (Molecular and Cellular Probes, (and see reference 4 below)
2. McGarrity, G. J. and Kotami, H. (1986) Exp. Cell. Res. 163:273-278
3. Langdale, J.A. and Malcolm, A.D.B. (1985) Gene 36:201-210; Malcolm, A.D.B. and Langdale, J.A. (1984) PCT WO 86/03782
4. Carrico, R.J., European Pat. Publ. 163,220.
5. Boguslawski, S.J. (1986) J. Immunol. Meth. 89:123.
6. Amasino, R.M. (1986) Anal. Biochem. 152: 304.

It is evident that the use of discrete, water suspensible microparticles as the solid carrier for the probe in the above-described methods enables the performance of a hybridization assay in significantly less time than the prior known methods.

The present invention has been particularly described and exemplified above. Obviously, many other variations and modifications of the invention may be made without departing from the spirit and scope hereof.

### Claims

1. A method for determining a particular polynucleotide sequence in a test medium containing single stranded nucleic acids, wherein either (i) the test medium is combined under hybridizing conditions with an immobilized form of a polynucleotide probe, the resulting immobilized phase is separated, and hybridized probe detected by binding of a reagent that selectively binds hybrids of the probe and the sequence to be determined but substantially not single stranded nucleic acids, or (ii) the test medium is treated to label the single stranded nucleic acids therein, an immobilized form of a polynucleotide probe is added under hybridizing conditions, the resulting immobilized phase is separated, and hybridized probe detected by measuring the label,

characterized in that the immobilized form of the polynucleotide probe comprises said probe attached to discrete, water suspensible microparticles whereby the hybridization and separation steps can be completed within about 30 minutes.

2. The method of claim 1 wherein a dispersion of said microparticles in the hybridization medium yields a fifty percent decrease in turbidity in no less than about 30 minutes.

3. The method of claim 1 wherein said microparticles have a specific gravity of from about 1.03 to about 1.08 grams/mL and have an average diameter of from about 0.1 to about 20 microns.

4. The method of claim 1 wherein the microparticle immobilized phase is separated by filtration or the microparticles are magnetic or magnetizable and the microparticle-immobilized phase is separated by application of a magnetic field.

5. The method of claim 1 wherein the hybrid-selective binding reagent is labeled with an enzymatically active group, a fluorescer, a chromophore, a luminescer, or a metal sol, or the label introduced to the sample nucleic acids is a ligand and is detectable by binding of a binding partner labeled with an enzymatically active group, a fluorescer, a chromophore, a luminescer, or a metal sol, whereby the hybridization, separation, and detection steps can all be completed within about 30 minutes.

6. The method of claim 1 wherein said anti-hybrid antibody reagent is selective for binding DNA RNA hybrids, one of the probe and the sequence to be determined being DNA and the other being RNA.

7. A reagent system for determining a particular polynucleotide sequence in a test medium by nucleic acid hybridization, comprising:

(1) an immobilized form of a polynucleotide probe having a single stranded base sequence to be determined, said probe being attached to discrete, water suspensible microparticles, and

(2) an anti-hybrid antibody reagent selective for binding hybrids formed between said probe and the sequence to be determined.

8. The reagent system of claim 7 wherein said microparticles have a specific gravity of from about 1.03 to about 1.08 grams/mL and have an average diameter of from about 0.1 to about 20 microns.

9. The reagent system of claim 7 wherein said anti-hybrid antibody reagent is selective for binding DNA RNA hybrids, one of the probe and the sequence to be determined being DNA and the other being RNA.

10. Use of the reagent system of claims 7 to 9 for the determination of a particular polynucleotide sequence in a test medium.

## HYBRIDIZATION TIME COURSE

FIG. 1

# COMPARISON WITH PRIOR METHODS

| METHOD | REFERENCE | TIME FROM INITIATION OF HYBRIDIZATION THROUGH____ | |
|---|---|---|---|
| | | SEPARATION STEP | MEASUREMENT STEP |
| PRESENT LATEX METHOD | — | 15 MIN | 20 MIN |
| SOLUTION HYBRIDIZATION (IMMOBILIZABLE PROBE) | (1) | 2.5 HRS | 2.5 HRS |
| SOLUTION HYBRIDIZATION (HYDROXYLAPATITE) | (2) | 65 MIN | 2-3 HRS |
| SEPHACRYL PARTICLES (SANDWICH) | (3) | 2-4 HRS | 3-5 HRS |
| DNA-CELLULOSE | (4) | 4-6 HRS | 6.5-8.5 HRS |
| NYLON BEADS | (5) | 18 HRS | 19.5 HRS |
| MICROPOROUS NYLON FILTER | (6) | 8-12 HRS | 3.5 DAYS |

# FIG. 2

0 288 737

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 605 815 (GENETICS INTENATIONAL INC.) <br> * Page 6, line 12 - page 7, line 18; page 20, lines 12-21; page 41, line 1 - page 49, line 11; figure 1 * | 1,4,10 | C 12 Q 1/68 <br> G 01 N 33/546 |
| X,P | WO-A-8 705 334 (ANGENICS INC.) <br> * Page 1, lines 20-29; page 6, lines 5-12; page 19, lines 7-29; page 29, line 2 - page 34, line 8; figures 1-5 * | 1-3,7-10 | |
| X | EP-A-0 159 719 (ENZO BIOCHEM INC.) <br> * Page 3, lines 5-10; page 5, lines 1-35; page 15, line 15 - page 16, line 9; page 32, line 2 - page 36, line 31; figure 1 * | 1,2,5-10 | |
| X | EP-A-0 192 168 (MOLECULAR DIAGNOSTICS INC.) <br> * Page 7, line 19 - page 8, line 20; page 13, line 25 - page 14, line 33; page 15, lines 24-36; page 24, line 2 - page 26, line 10; figure * | 1,2,5-10 | TECHNICAL FIELDS SEARCHED (Int. Cl.4) <br><br> C 12 Q <br> G 01 N <br> C 07 H |
| X | EP-A-0 152 886 (MOLECULAR DIAGNOSTICS INC.) <br> * Page 4, lines 4-10; page 13, line 1 - page 15, line 5 * | 1,2,10 | |
| X | EP-A-0 209 702 (MILES LABORATORIES INC.) <br> * Page 3, lines 15-33; page 5, line 25 - page 6, line 32; page 8, line 30 - page 11, line 20; page 16, line 2 - page 19, line 22; page 23, line 20 - page 25, line 23; page 32, line 2 - page 34, line 3 * | 1-10 | |

-/-

The present search report has been drawn up for all claims

| Place of search | Date of completion of the search | Examiner |
|---|---|---|
| THE HAGUE | 14-07-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 146 039 (MILES LABORATORIES INC.) * Page 3, lines 7-34; page 5, line 36 - page 9, line 7; page 12, lines 4-28; page 21, lines 2-29; page 26, line 26 - page 27, line 9; page 32, example I; page 39, example II; page 49, line 1 - page 51, line 22 * | 1-10 | |
| X | EP-A-0 146 815 (MILES LABORATORIES INC.) * Page 5, line 17 - page 6, line 34; page 27, lines 2-20; page 62, line 1 - page 65, line 23; figures 2-5 * | 1-10 | |
| X | EP-A-0 144 913 (MILES LABORATORIES INC.) * Page 3, line 22 - page 6, line 13; page 10, line 23 - page 12, line 16; page 26, line 2 - page 34, line 18; page 51, line 1 - page 54, line 21 * | 1-10 | TECHNICAL FIELDS SEARCHED (Int. Cl.4) |
| X | EP-A-0 001 223 (HOFFMAN-LA ROCHE & CO.) * Page 3, line 27 - page 4, line 14; page 23, lines 1-11 * | 3,8,10 | |

The present search report has been drawn up for all claims

| Place of search | Date of completion of the search | Examiner |
|---|---|---|
| THE HAGUE | 14-07-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                  

& : member of the same patent family, corresponding document

EPO FORM 1503 03.82 (P0401)